(19) Europäisches Patentamt
European Patent Office
Office européen des brevets

(11) Numéro de publication : **0 315 540 B1**

# (12) FASCICULE DE BREVET EUROPEEN

(45) Date de publication du fascicule du brevet : **29.04.92 Bulletin 92/18**

(51) Int. Cl.$^5$ : **C07H 17/08,** A61K 31/70, A61K 7/48

(21) Numéro de dépôt : **88402771.5**

(22) Date de dépôt : **03.11.88**

## (54) Esters rétinoiques de macrolides, leur procédé de préparation et compositions pharmaceutiques et cosmétiques les contenant.

(30) Priorité : **04.11.87 LU 87041**

(43) Date de publication de la demande : **10.05.89 Bulletin 89/19**

(45) Mention de la délivrance du brevet : **29.04.92 Bulletin 92/18**

(84) Etats contractants désignés : **AT BE CH DE ES FR GB GR IT LI NL SE**

(56) Documents cités :
**FR-A- 2 598 420**
**GB-A- 2 175 303**
**US-A- 4 056 616**
**J. MED. CHEM., vol. 25, 1982, pages 81-84, American Chemical Society; S.C. WELCH et al.: "Syntheses and activities of antioxidant derivatives of retinoic acid"**

(73) Titulaire : **L'OREAL**
**14, Rue Royale**
**F-75008 Paris (FR)**

(72) Inventeur : **Philippe, Michel**
**3, rue de l'Aubépine**
**F-92160 Antony (FR)**
Inventeur : **Sebag, Henri**
**26, rue Erlanger**
**F-75016 Paris (FR)**

(74) Mandataire : **Stalla-Bourdillon, Bernard et al**
**CABINET NONY & CIE 29, rue Cambacérès**
**F-75008 Paris (FR)**

**EP 0 315 540 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des esters rétinoïques de macrolides, leur procédé de préparation et des compositions pharmaceutiques et cosmétiques les contenant dans le traitement de diverses dermatoses, notamment dans le traitement de l'acné.

Plus particulièrement, les esters rétinoïques selon l'invention sont destinés au traitement des dermatoses infectieuses ou non.

Les esters rétinoïques selon l'invention apportent une solution satisfaisante dans le traitement de l'acné, dans la mesure où il s'est avéré que ceux-ci avaient une action sur propionibacterium acnes, principal germe responsable des phénomènes d'inflammation de la peau.

Les esters rétinoïques selon l'invention ont du fait de leur structure un caractère lipophile prononcé, ce qui facilite une meilleure pénétration à travers l'épiderme.

La présente invention a pour objet, à titre de produit industriel nouveau, des esters d'acides rétinoïques all trans et 13-cis de macrolides repondant à la formule générale suivante:

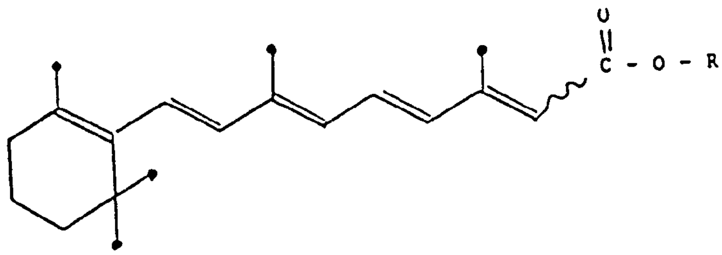

(I)

dans laquelle:

R représente un radical dérivé d'un macrolide à l'exclusion de l'érythromycine A,

et les mélanges et sels desdits esters.

Selon l'invention, les macrolides sont de préférence la roxythromycine, l'oléandomycine, la josamycine et les spiramycines (I), (II) et (III).

A - Les esters rétinoïques de roxythromycine peuvent être représentés par la formule:

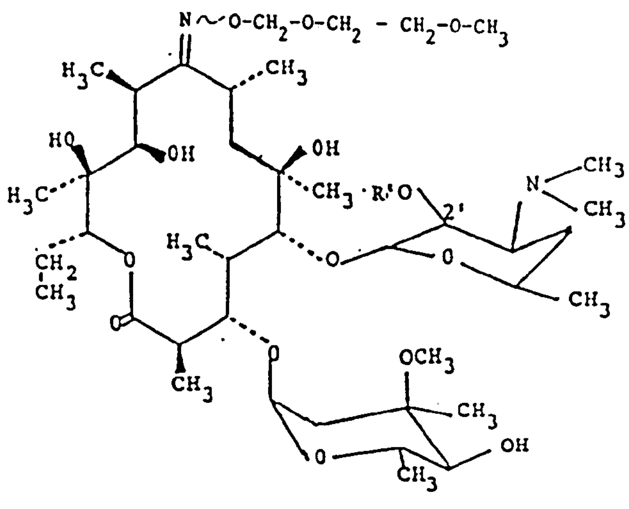

(II)

dans laquelle:

R' représente le radical acyle suivant:

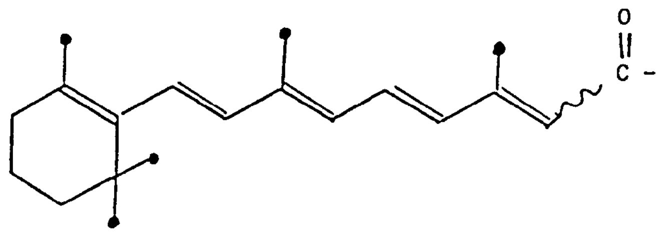

(I')

Ces esters rétinoïques de roxythromycine sont ceux en position 2′.

B - Les esters rétinoïques d'oléandomycine peuvent être représentés par la formule suivante:

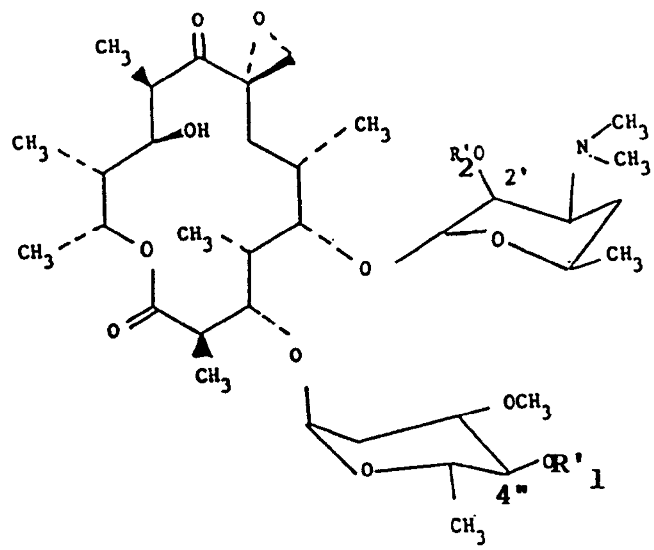

(III)

dans laquelle:

$R'_1$ ou $R'_2$ représente R′ ou un atome d'hydrogène, étant entendu que l'un au moins représente R′, R′ ayant la même signification que ci-dessus.

Ces esters rétinoïques d'oléandomycine sont ceux en position 2′ et/ou 4″ mais ils peuvent se présenter sous forme d'un mélange.

C - Les esters rétinoïques de josamycine peuvent être représentés par la formule suivante:

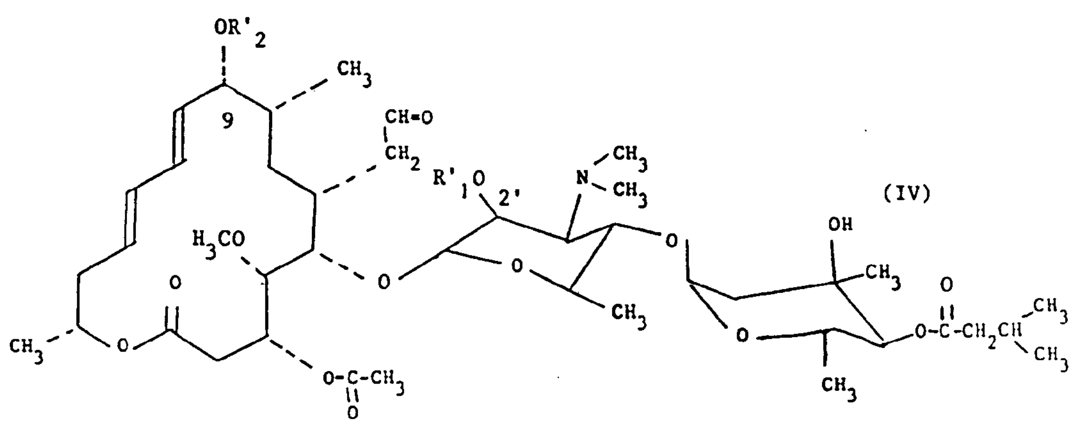

(IV)

dans laquelle:

$R'_1$ ou $R'_2$ représente R′ ou un atome d'hydrogène, étant entendu que l'un au moins représente R′, R′

ayant la même signification que ci-dessus.

Ces esters rétinoïques de josamycine sont ceux en position 9 et/ou 2′ mais il peuvent se présenter sous forme d'un mélange.

D - Les esters rétinoïques des spiramycines peuvent être représentés par la formule suivante:

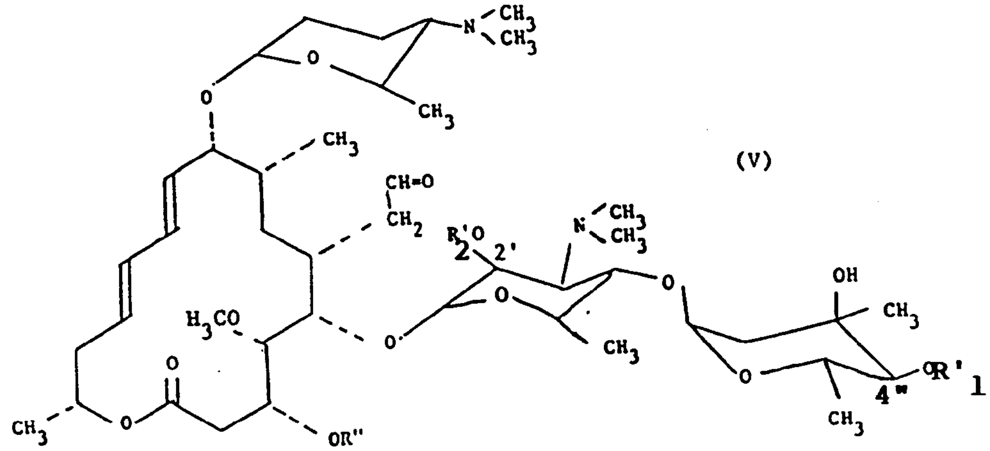

(V)

dans laquelle:

R′$_1$ ou R′$_2$ représente R′ ou un atome d'hydrogène étant entendu que l'un au moins représente R′,

R′ ayant la même signification que ci-dessus

et R″ représente un atome d'hydrogène (spiramycine I), un radical acétyl (spiramycine II) ou un radical propionyl (spiramycine III).

Ces esters des spiramycines (I), (II) et (III) sont ceux en position 2′ et/ou 4″, ceux-ci pouvant éventuellement se présenter sous forme d'un mélange.

Les esters rétinoïques de formule (I) selon l'invention sont de préférence les suivants:

O-rétinoyl (13-cis)-2′-roxythromycine
O-rétinoyl (all trans)-2′-oléandomycine
O-rétinoyl (all trans)-4″-oléandomycine
O-rétinoyl (all trans)-9-josamycine
O-rétinoyl (all trans)-2′-josamycine
O-rétinoyl (13-cis)-2′-spiramycine (I), (II) et (III),
O-rétinoyl (all-trans)-3-spiramycine (I), (II) et (III),
O-rétinoyl (all-trans)-4″-spiramycine (I), (II) et (III),
O-rétinoyl (13-cis)-9-josamycine.

La présente invention a également pour objet le procédé de préparation des esters rétinoïques all trans et 13-cis de formule (I) ci-dessus.

Différents procédés d'estérification peuvent être utilisés mais de préférence cette estérification est réalisée en milieu solvant organique anhydre, de préférence dans le tétrahydrofuranne seul ou en mélange avec un autre solvant organique comme la pyridine, en faisant réagir un excès d'anhydride mixte de l'acide rétinoïque all trans ou 13-cis préparé in situ, par exemple à partir de chloroformiate d'éthyle et d'acide rétinoïque all trans ou 13-cis, avec un macrolide tel que ceux énumérés ci-dessus, sous forme de base, en présence d'une base organique ou minérale comme la pyridine et/ou l'hydrogénocarbonate de sodium et/ou la triéthylamine.

La présente invention a également pour objet des compositions pharmaceutiques administrables par voie topique, orale, parentérale ou rectale ainsi que des compositions à caractère cosmétique pour le traitement de diverses dermatoses, notamment l'acné, ces compositions se présentant sous forme anhydre et contenant au moins un ester rétinoïque de formule (I) telle que définie ci-dessus, à une concentration comprise entre 0,001 et 10% mais de préférence entre 0,01 et 1% en poids par rapport au poids total de la composition.

Pour la préparation des compositions selon l'invention contenant, comme constituant actif, au moins un ester rétinoïque de formule (I), on peut faire appel à des véhicules et adjuvants décrits dans la littérature pour la pharmacie, la cosmétique et les domaines apparentés.

Pour la préparation des solutions, on peut utiliser par exemple un (ou des) solvant(s) organique(s) acceptable(s) d'un point de vue physiologique.

Les solvants organiques acceptables sont pris notamment dans le groupe constitué par l'acétone, l'alcool

isopropylique, les triglycérides d'acides gras, les esters d'alkyle en $C_1$-$C_4$ d'acides à courte chaîne, les éthers du polytétrahydrofuranne et les silicones comme les cyclométhicones.

Les compositions selon l'invention peuvent également renfermer un agent épaississant tel qu'un dérivé de la cellulose à raison de 0,5 à 20% en poids par rapport au poids total de la composition.

Les compositions selon l'invention peuvent en outre contenir en association avec au moins un ester selon l'invention, au moins un autre agent anti-acnéique connu.

On peut, si nécessaire, ajouter un adjuvant usuel pris dans le groupe formé par les agents anti-oxydants, les agents conservateurs, les parfums et les colorants.

Parmi les anti-oxydants utilisables, on citera par exemple la t-butylhydroxyquinone, le butylhydroxyanisole, le butylhydroxytoluène et l'$\alpha$-tocophérol et ses dérivés.

Les transformations pharmacologiques et galéniques des composés selon l'invention s'effectuent de façon connue.

Les formes galéniques peuvent être pour la voie topique des crèmes, des laits, des gels, des lotions plus ou moins épaissies, des lotions portées par des tampons, des pommades, des sticks ou bien des formulations aérosols se présentant sous forme de sprays ou de mousses.

Les compositions par voie orale peuvent se présenter sous forme de comprimés, de gelules, de dragées, de sirops, de suspensions, d'émulsions, de poudres, de granulés ou de solutions.

Les compositions peuvent également se présenter sous forme de suppositoires.

Le traitement de l'acné à l'aide des compositions topiques selon l'invention consiste à appliquer, deux ou trois fois par jour, une quantité suffisante sur les zones de la peau à traiter et ceci pendant une période de temps de 6 à 30 semaines et de préférence de 12 à 24 semaines.

Les compositions selon l'invention peuvent également être utilisées à titre préventif, c'est-à-dire sur les zones de peau susceptibles d'être atteintes d'acné.

On va maintenant donner à titre d'illustration plusieurs exemples de préparation des esters rétinoïques de formule (I) ainsi que plusieurs exemples de compositions pharmaceutiques ou cosmétiques dans le traitement des dermatoses, notamment de l'acné.

EXEMPLE 1

Préparation du O-rétinoyl (all trans)-9-josamycine

Dans un ballon, sous atmosphère inerte, on dissout 5g (16,6mmoles) d'acide rétinoïque (all trans) dans 35ml de tétrahydrofuranne anhydre; le mélange réactionnel est refroidi à 0°C, puis on verse 2,3ml de triéthylamine et 1,6ml (16,6mmoles) de chloroformiate d'éthyle. La solution est agitée 5 minutes et on ajoute 20ml de pyridine anhydre puis 5,8g (7mmoles) de josamycine préalablement dissous dans 150ml de tétrahydrofuranne. Le mélange réactionnel est alors laissé sous agitation pendant 10 heures, en laissant remonter à température ambiante (chromatographie sur couche mince de gel de silice: chlorure de méthylène (90)/méthanol (10)). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C) en utilisant l'éluant : acétate d'éthyle (7)/hexane (3) pour aboutir à l'isolement de 4,7g (60% de rendement) de O-rétinoyl (all trans)-9- josamycine et une trace de son isomère 13-cis.

F = 110°C (hexane/acétate d'éthyle).

Microanalyse: $C_{62}H_{95}N\ O_{16}$ , 1,5 $H_2O$ ; PM= 1137,5

| | C | H | N |
|---|---|---|---|
| Calculé %: | 65,46 | 8,68 | 1,23 |
| Trouvé %: | 65,26 | 8,55 | 1,19 |

Infra-rouge : bande à 1735cm$^{-1}$ (ester).

R.M.N. du $^{13}C$ ($CDCl_3$, réf. interne T.M.S.).

Effets $\gamma$ négatifs en 8 (-2,2ppm) et 10 (-1,3ppm) indiquent la position de l'ester en 9.

EXEMPLE 2

Préparation du O-rétinoyl(all trans)-2′-josamycine

Dans un ballon, sous atmosphère inerte on dissout 5g (16,6 mmoles) d'acide rétinoique (all trans) dans 35 ml de tétrahydrofuranne anhydre; le mélange réactionnel est refroidi à 0°C puis on verse 3 ml (38 mmoles) de pyridine anhydre et 1,6 ml (16,6 mmoles) de chloroformiate d'éthyle ; la solution est agitée 5 minutes et on ajoute 25 ml de pyridine anhydre puis 5,8 g (7 mmoles) de josamycine préalablement dissous dans 150 ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 7,5 %). La solution est versée sur 100 ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant : acétate d'éthyle (7)/hexane (3) pour aboutir à l'isolement notamment de 1,5 g (20 % de rendement) de O-rétinoyl (all trans)-2′-josamycine et une trace de son isomère 13 cis.

F = 130°C (acétate d'éthyle/hexane)

Microanalyse: $C_{62}H_{95}N\ O_{16}$ , 1,5 $H_2O$ ; M= 1137,5

| | | C | H | N |
|---|---|---|---|---|
| Calculé % | : | 65,49 | 8,68 | 1,23 |
| Trouvé % | : | 65,76 | 8,21 | 1,22 |

R.M.N. du $^{13}C$ ($CDCl_3$, réf. interne T.M.S.)

Effets $\gamma$ négatifs en 1′ (-2,1 ppm) et 3′ (-1,62 ppm) indiquent la position de l'ester en 2′.

EXEMPLE 3

Préparation des O-rétinoyl(all trans)-2′ oléandomycine et O-rétinoyl (all trans)-4″-oléandomycine.

Dans un ballon, sous atmosphère inerte on dissout 5g (16,6 mmoles) d'acide rétinoique (all trans) dans 35 ml de tétrahydrofuranne anhydre. Le mélange réactionnel est refroidi à 0°C puis on verse 2,4 ml (16,7 mmoles) de triéthylamine et 1,6 ml (16,6 mmoles) de chloroformiate d'éthyle ; la solution est agitée 5 minutes et on ajoute 25 ml de pyridine anhydre puis 4,1g (6 mmoles) d'oléandomycine préalablement dissous dans 200 ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 10 %). La solution est versée sur 100 ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant : acétate d'éthyle (7)/hexane (3) pour aboutir à l'isolement de 3,2g (55 % de rendement) de O-rétinoyl (all trans)-4″-d'oléandomycine majoritaire et de O-rétinoyl (all trans)-2′-oléandomycine minoritaire.

Microanalyse: $C_{55}H_{87}N\ O_{13}$ , 1,5 $H_2O$ ; M= 997

| | | C | H | N |
|---|---|---|---|---|
| Calculé % | : | 66,24 | 9,09 | 1,4 |
| Trouvé % | : | 66,23 | 8,96 | 1,50 |

R.M.N. du $^{13}C$ ($CDCl_3$, réf. interne T.M.S.)

effets $\gamma$ négatifs en 3″ (-2,5 ppm) et 5″ (-1,5ppm) indiquent la position de l'ester en 4″ majoritaire.

EXEMPLE 4

Préparation du O-rétinoyl(13 cis)-2′-roxythromycine

Dans un ballon, sous atmosphère inerte, on dissout 5g (16,6 mmoles) d'acide rétinoique (13-cis) dans 35

ml de tétrahydrofuranne anhydre le mélange réactionnel est refroidi à 0°C puis on verse 2,5 ml de triéthylamine et 1,6 ml (16,6 mmoles) de chloroformiate d'éthyle. La solution est agitée 5 minutes et on ajoute 25 ml de pyridine anhydre puis 5g (6 mmoles) de roxythromycine préalablement dissous dans 150 ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 10 %). La solution est versée sur 120 ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant : acétate d'éthyle (7)/hexane (3) pour aboutir à l'isolement de 4 g (60 % de rendement) de O-rétinoyl (13 cis)-2'-roxythromycine et une trace de son isomère all trans.

F = 117°C (hexane/acétate d'éthyle)

Microanalyse: $C_{61}H_{102}N\ O_{16}$ , $3H_2O$ ; PM= 1137,6

| | | | C | H | N |
|---|---|---|---|---|---|
| Calculé | % | : | 62,43 | 9,28 | 2,39 |
| Trouvé | % | : | 62,39 | 8,65 | 2,21 |

R.M.N. du $^{13}C$ ($CDCl_3$, réf. interne T.M.S.)

Effets $\gamma$ négatifs en 1'(-2,4 ppm) et 3'(-2,1 ppm) indiquent la position de l'ester en 2'.

Les autres esters énumérés à la page 4 peuvent être préparés selon les mêmes modes opératoires que décrits ci-dessus aux exemples 1 à 4.

COMPOSITIONS PHARMACEUTIQUES ET COSMETIQUES

A. GEL POUR LE TRAITEMENT TOPIQUE DE L'ACNE

- Hydroxypropyl cellulose.................... 1g
- Butylhydroxytoluène........................ 0,05g
- O-rétinoyl (all trans)-9-josamycine......... 0,5g
- Isopropanol q.s.p.......................... 100g

Dans cet exemple, le composé actif peut être remplacé par une quantité identique d'O-rétinoyl (13 cis)-2'-roxythromycine.

B. LOTION POUR LE TRAITEMENT TOPIQUE DE L'ACNE

- Butylhydroxytoluène........................ 0,05g
- O-rétinoyl (all trans)-9-josamycine......... 1g
- Triglycérides d'acides gras en $C_8$-$C_{12}$ q.s.p.......................... 100g

Dans cet exemple le composé actif peut être remplacé par une quantité identique d'O-rétinoyl (all trans)-2'-josamycine.

C. STICK POUR LE TRAITEMENT TOPIQUE DE L'ACNE

- Vaseline blanche.......................... 52g
- Huile de vaseline......................... 15g
- Paraffine raffinée........................ 32g
- O-rétinoyl (all trans)-9-josamycine......... 1g

Dans cet exemple le composé actif peut être remplacé par une quantité identique d'O-rétinoyl (13 cis)-2′-roxythromycine ou du mélange d'O-rétinoyl (all trans)-2′-oléandomycine et d'O-rétinoyl (all trans)-4″-oléandomycine.

## Revendications

1. Esters d'acide rétinoïques all trans et 13-cis de macrolides repondant à la formule suivante:

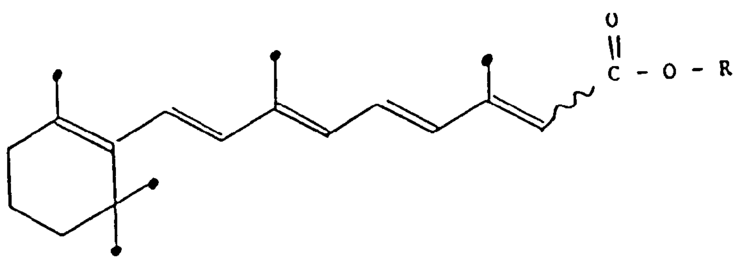

(I)

dans laquelle:

R représente un radical dérivé d'un macrolide à l'exclusion de l'érythromycine A,

et les mélanges et sels desdits esters.

2. Esters selon la revendication 1, caractérisés par le fait que le reste de macrolide est choisi parmi la roxythromycine, l'oléandomycine, la josamycine et les spiramycines (I), (II) et (III).

3. Esters selon les revendications 1 et 2, caractérisés par le fait qu'ils sont de préférence pris dans le groupe constitué par:

O-rétinoyl (13-cis)-2′-roxythromycine
O-rétinoyl (all trans)-2′-oléandomycine
O-rétinoyl (all trans)-4″-oléandomycine
O-rétinoyl (all trans)-9-josamycine
O-rétinoyl (all trans)- 2′-josamycine
O-rétinoyl (13-cis)-2′-spiramycine (I), (II) et (III),
O-rétinoyl (all-trans)-3-spiramycine (I), (II) et (III),
O-rétinoyl (all-trans)-4″-spiramycine (I), (II) et (III)
O-rétinoyl (13-cis)-9-josamycine.

4. Procédé de préparation des esters rétinoïques selon la revendication 1, caractérisé par le fait qu'il consiste à faire réagir en milieu solvant organique anhydre, un excès d'anhydride mixte de l'acide rétinoïque all trans ou 13-cis, préparé in situ, avec un macrolide choisi parmi: la roxythromycine, l'oléandomycine, la josamycine ou la spiramycine (I), (II) et (III), sous forme de base, en présence d'une base organique ou minérale.

5. Procédé selon la revendication 4, caractérisé par le fait que le solvant organique anhydre est le tétrahydrofuranne seul ou en mélange avec de la pyridine.

6. Procédé selon la revendication 4, caractérisé par le fait que la base organique ou minérale est la pyridine et/ou l'hydrogénocarbonate de sodium et/ou la triéthylamine.

7. Composition pharmaceutique ou cosmétique pour le traitement de diverses dermatoses, notamment de l'acné, caractérisée par le fait qu'elle contient dans un véhicule anhydre, en tant que composé actif, au moins un ester rétinoïque selon les revendications 1 à 3 ou obtenu selon l'une quelconque des revendications 4 à 6.

8. Composition selon la revendication 7, caractérisée par le fait qu'elle contient de 0,001 à 10% en poids et de préférence de 0,01 à 1% d'ester rétinoïque.

9. Composition selon l'une quelconque des revendications 7 et 8, caractérisée par le fait que le véhicule est l'acétone, l'alcool isopropylique, les triglycérides d'acides gras, les esters d'alkyle en $C_1$-$C_4$ d'acides à courte chaîne, les éthers de polytétrahydrofuranne, les silicones et leurs mélanges.

10. Composition selon l'une quelconque des revendications 7 à 9, caractérisée par le fait qu'elle contient en outre un agent épaississant tel qu'un dérivé de cellulose en une proportion de 0,5 à 20% en poids par rapport au poids total de la composition.

11. Composition selon l'une quelconque des revendications 7 à 10, caractérisée par le fait qu'elle contient également un agent anti-oxydant, un agent conservateur, un parfum, un colorant ou un autre agent anti-acnéique.

## Claims

1. All-trans- and 13-cis-retinoic acid esters of macrolides corresponding to the following formula:

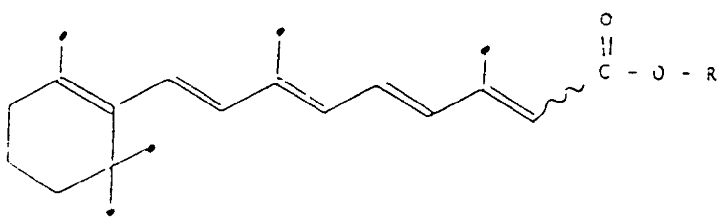

(I)

in which:

R denotes a radical derived from a macrolide excluding erythromycin A,

and the mixtures and salts of the said esters.

2. Esters according to Claim 1, characterized in that the macrolide residue is chosen from roxythromycin, oleandomycin, josamycin and spiramycins (I), (II) and (III).

3. Esters according to Claims 1 and 2, characterized in that they are preferably selected from the group consisting of:

2′-O-(13-cis-retinoyl)roxythromycin
2′-O-(all-trans-retinoy1)oleandomycin
4″-O-(all-trans-retinoyl)oleandomycin
9-O-(all-trans-retinoyl)josamycin
2′-O-(all-trans-retinoyl)josamycin
2′-O-(13-cis-retinoyl)spiramycin (I), (II) and (III)
3-O-(all-trans-retinoyl)spiramycin (I), (II) and (III)
4″-O-(all-trans-retinoyl)spiramycin (I), (II) and (III)
9-O-(13-cis-retinoyl)josamycin.

4. Process for preparing the retinoic esters according to Claim 1, characterized in that it consists in reacting, in an anhydrous organic solvent medium, an excess of mixed anhydride of all-trans- or 13-cis-retinoic acid, prepared in situ, with a macrolide chosen from roxythromycin, oleandomycin, josamycin or spiramycin (I), (II) and (III), in base form, in the presence of an organic or inorganic base.

5. Process according to Claim 4, characterized in that the anhydrous organic solvent is tetrahydrofuran, alone or mixed with pyridine.

6. Process according to Claim 4, characterized in that the organic or inorganic base is pyridine and/or sodium hydrogen carbonate and/or triethylamine.

7. Pharmaceutical or cosmetic composition for the treatment of various dermatoses, in particular acne, characterized in that it contains, as active compound, at least one retinoic ester according to Claims 1 to 3 or obtained according to any one of Claims 4 to 6, in an anhydrous vehicle.

8. Composition according to Claim 7, characterized in that it contains from 0.001 to 10% by weight, and preferably from 0.01 to 1%, of retinoic ester.

9. Composition according to either of Claims 7 and 8, characterized in that the vehicle is acetone, isopropyl alcohol, fatty acid triglycerides, $C_1$-$C_4$ alkyl esters of short-chain acids, polytetrahydrofuran ethers, silicones and mixtures thereof.

10. Composition according to any one of Claims 7 to 9, characterized in that it contains, in addition, a thickening agent such as a cellulose derivative, in a proportion of 0.5 to 20% by weight relative to the total weight of the composition.

11. Composition according to any one of Claims 7 to 10, characterized in that it also contains an antioxidant, a preservative, a fragrance, a colouring or another anti-acne agent.

## Patentansprüche

1. Makrolidester der all-trans- und 13-cis-Retinoesäure folgender Formel:

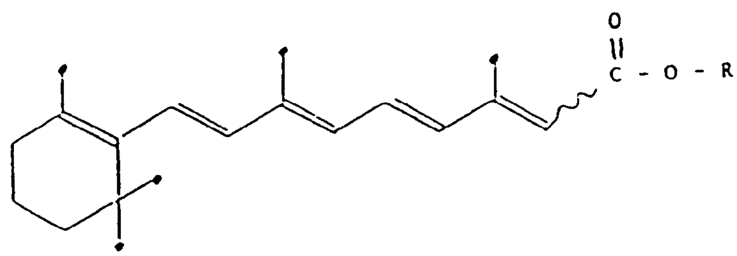

(I)

worin

R für einen von einem Makrolid mit Ausnahme von Erythromycin A abgeleiteten Rest steht,

und die Mischungen und Salze dieser Ester.

2. Ester nach Anspruch 1, dadurch gekennzeichnet, daß der Makrolidrest ausgewählt ist unter Roxythromycin, Oleandomycin, Josamycin und den Spiramycinen (I), (II) und (III).

3. Ester nach den Ansprüchen 1 und 2, dadurch gekennzeichnet, daß sie vorzugsweise ausgewählt sind unter:

O-Retinoyl-(13-cis)-2′-roxythromycin,
O-Retinoyl(-all-trans)-2′oleandomycin,
O-Retinoyl-(all-trans)-4″-oleandomycin,
O-Retinoyl-(all-trans)-9-josamycin,
O-Retinoyl-(all-trans)-2′-josamycin,
O-Retinoyl-(13-cis)-2′-spiramycin (I), (II) und (III),
O-Retinoyl-(all-trans)-3-spiramycin (I), (II) und (III),
O-Retinoyl-(all-trans)-4″-spiramycin (I), (II) und (III),
O-Retinoyl-(13-cis)-9-josamycin.

4. Verfahren zur Herstellung der Retinoesäureester nach Anspruch 1, dadurch gekennzeichnet, daß man in wasserfreiem organischem Lösungsmittelmilieu einen Überschuß eines in situ hergestellten gemischten Anhydrids der all-trans- oder 13-cis-Retinoesäure mit einem Makrolid, ausgewählt unter Roxythromycin, Oleandomycin, Josamycin oder Spiramycin (I), (II) und (III) in Form der Base in Anwesenheit einer organischen oder mineralischen Base reagieren läßt.

5. Verfahren nach Anspruch 4, dadurch gekennzeichnet, daß es sich bei dem wasserfreien organischen Lösungsmittel um Tetrahydrofuran, allein oder mit Pyridin gemischt, handelt.

6. Verfahren nach Anspruch 4, dadurch gekennzeichnet, daß es sich bei der organischen oder mineralischen Base um Pyridin und/oder Natriumhydrogencarbonat und/oder Triethylamin handelt.

7. Pharmazeutisches oder kosmetisches Mittel zur Behandlung verschiedener Dermatosen, insbesondere Akne, dadurch gekennzeichnet, daß es in einem wasserfreien Träger als aktive Verbindung mindestens einen Retinoesäureester, gemäß den Ansprüchen 1 bis 3 oder erhalten gemäß einem der Ansprüche 4 bis 6, enthält.

8. Mittel nach Anspruch 7, dadurch gekennzeichnet, daß es 0,001 bis 10 Gew.-% und vorzugsweise 0,01 bis 1 Gew.-% Retinoesäureester enthält.

9. Mittel nach einem der Ansprüche 7 und 8, dadurch gekennzeichnet, daß es sich bei dem Träger um Aceton, i-Propylalkohol, Fettsäuretriglyceride, Alkylester kurzkettiger $C_1$-$C_4$-Säuren, Polytetrahydrofuranäther, Silicone und Mischungen davon handelt.

10. Mittel nach einem der Ansprüche 7 bis 9, dadurch gekennzeichnet, daß es außerdem ein Verdickungsmittel, wie ein Cellulosederivat, in einer Menge von 0,5 bis 20 Gew.-%, bezogen auf das Gesamtgewicht des Mittels, enthält.

11. Mittel nach einem der Ansprüche 7 bis 10, dadurch gekennzeichnet, daß es auch ein Antioxidationsmttel, ein Konservierungsmittel, ein Parfum, einen Farbstoff oder ein anderes Antiaknemittel enthält.